(19) Europäisches Patentamt
European Patent Office
Office européen des brevets

(11) Veröffentlichungsnummer: **0 106 080**
**B1**

(12) # EUROPÄISCHE PATENTSCHRIFT

(45) Veröffentlichungstag der Patentschrift: **28.01.87**

(21) Anmeldenummer: **83108383.7**

(22) Anmeldetag: **25.08.83**

(51) Int. Cl.[4]: **C 07 D 333/20**, C 07 D 277/28, C 07 C 161/00, C 07 C 143/82, C 07 D 295/08, A 61 K 31/38, A 61 K 31/425, A 61 K 31/155, A 61 K 31/63

(54) **Heterocyclische Verbindungen, Verfahren zu ihrer Herstellung und diese Verbindungen enthaltende Arzneimittel.**

(30) Priorität: **20.10.82 DE 3238867**

(43) Veröffentlichungstag der Anmeldung: **25.04.84 Patentblatt 84/17**

(45) Bekanntmachung des Hinweises auf die Patenterteilung: **28.01.87 Patentblatt 87/5**

(84) Benannte Vertragsstaaten: **AT BE CH DE FR GB IT LI LU NL SE**

(56) Entgegenhaltungen:
**EP - A - 0 002 930**
**DE - A - 2 821 409**
**DE - A - 3 008 056**
**GB - A - 538 822**

**CHEMICAL ABSTRACTS, Band 96, Nr. 25, 21. Juni 1982, Seite 746, Nr. 217853x, Columbus, Ohio, US**
**CHEMICAL ABSTRACTS, Band 97, Nr. 5, 2. August 1982, Seite 108, Nr. 33922h, Columbus, Ohio, US, T. TAKAGI u.a.: "*Effect of a new potent H2-blocker, 3-(((2-((diaminomethylene)aminol)-4-thiazolyl)methyl)thio)-N2-sulfamoylpropionamidine (YM-11170) on gastric secretion induced by histamine and food in conscious dogs*"**

(73) Patentinhaber: **LUDWIG HEUMANN & CO GMBH, Heideloffstrasse 18-28 Postfach 2260, D-8500 Nürnberg (DE)**

(72) Erfinder: **Szelenyi, Istvan, Dr., Brahmsstrasse 16, D-8501 Schwaig (DE)**
Erfinder: **Postius, Stefan, Dr., Unschlittplatz 1, D-8500 Nürnberg (DE)**
Erfinder: **Schickaneder, Helmut, Dr., Moosäcker 25, D-8501 Eckental (DE)**
Erfinder: **Hansen, Herbert, Wolkersdorfer Berg 11, D-8540 Schwabach 7 (DE)**

(74) Vertreter: **Kraus, Walter, Dr. et al, Patentanwälte Kraus, Weisert & Partner Thomas-Wimmer-Ring 15, D-8000 München 22 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft neue Amidin-Derivate mit einer Wirkung auf Histamin-$H_2$-Rezeptoren, Verfahren zu ihrer Herstellung und Arzneimittel, die diese Verbindungen enthalten, sowie schliesslich die Verwendung dieser Verbindungen in der Therapie.

Die erfindungsgemäss herstellbaren Verbindungen zeigen aufgrund ihrer $H_2$-antagonistischen Aktivität eine Hemmung der Magensäuresekretion, wenn diese durch Histamin-Agonisten stimuliert wird (Ash und Schild «Brit. J. Pharmacol. Chemother.» 27, 427 (1966) und Black et al «Nature» 236, 385 (1972). Die pharmakologische Aktivität dieser Verbindungen, wie sie genauer weiter unten beschrieben werden, kann nach einer modifizierten Methode gemäss der DE-OS 2734070 beim perfundierten Rattenmagen gezeigt werden. Weiterhin kann die $H_2$-antagonistische Wirkung an wachen Heidenhain-Pouch-Hunden nach der Methode von Black et al «Nature» 236, 385 (1971) gezeigt werden. Die neuen Verbindungen antagonisieren weiterhin die Histaminwirkung auf die Kontraktionsfrequenz des isolierten rechten Atriums des Meerschweinchens, aber sie beeinflussen nicht histamininduzierte Kontraktionen des isolierten, glatten gastrointestinalen Muskels, wenn diese durch $H_1$-Agonisten hervorgerufen werden.

Nachdem Hemmstoffe für Histamin-$H_2$-Rezeptoren sowohl bezüglich der basalen Magensäuresekretion als auch der durch Gastrin, Histamin, Methacholin oder Nahrung induzierten Magensäuresekretion Hemmwirkung besitzen, können sie für die Behandlung von peptischen Ulcera, die duch übermässige Sekretion von Magensäure verursacht sind, und bei der Therapie hyperacider Gastritis verwendet werden.

In Chem. Abstr. 97 (1982) 33922h wird 3-[[[2-[(Diaminomethylen) amino]-4-thiazolyl] methyl]thio]-$N^2$-sulfamoylpropionamidin beschrieben. Nach den Angaben dieser Druckschrift handelt es sich bei dieser Verbindung um einen neuen potenten $H_2$-Blocker. Die DE-A 2821409 beschreibt bestimmte Aminoalkylthiophenverbindungen mit $H_2$-antagonistischer Wirksamkeit. Auch in der EP-A 2930 werden bestimmte Aminderivate mit $H_2$-antagonistischer Wirkung beschrieben.

Der Erfindung liegt die Aufgabe zugrunde, neue Hemmstoffe für Histamin-$H_2$-Rezeptoren mit verbesserter Wirksamkeit zur Verfügung zu stellen. Diese Aufgabe wird durch die Erfindung gelöst.

Gegenstand der Erfindung sind heterocyclische Verbindungen der allgemeinen tautomeren Formeln I:

$$R^1R^2NAlk-Q-XY(CH_2)_m-\overset{\overset{NH}{\|}}{C}-NH-\overset{\overset{O}{\|}}{\underset{\underset{O}{\|}}{S}}-R^3 \rightleftharpoons R^1R^2NAlk-Q-XY(CH_2)_m-\overset{\overset{NH_2}{|}}{C}=N-\overset{\overset{O}{\|}}{\underset{\underset{O}{\|}}{S}}-R^3 \quad (I)$$

in denen $R^1$ für lineares $C_{1-6}$Alkyl und $R^2$ lineares $C_{1-6}$Alkyl steht oder wobei $R^1$ und $R^2$ zusammen mit dem Stickstoffatom, an das sie gebunden sind, einen 5- oder 6gliedrigen Ring bilden;

Alk für eine geradkettige Alkylenkette mit 1 bis 6 Kohlenstoffatomen steht;

Q für einen Thiophenring steht, dessen Einarbeitung in den Rest des Moleküls durch Bindungen in 2- und 5-Stellung oder in 2- und 4-Stellung erfolgt ist, oder wobei Q für einen Benzolring steht, dessen Einarbeitung in den Rest des Moleküls durch Bindungen in 1- und 3-Stellung erfolgt ist;

X in dem Fall, das Q die Bedeutung Thiophen hat, für Methylen, Y für Schwefel steht und m den Wert 2 oder 3 hat; X in dem Fall, dass Q die Bedeutung Benzol hat, für Sauerstoff, Y für eine Einfachbindung steht und m den Wert 3 oder 4 hat; $R^3$ für eine Aminogruppe oder eine p-Aminophenylgruppe steht;

sowie die physiologisch annehmbaren Salze davon.

Eine bevorzugte Gruppe von erfindungsgemässen Verbindungen ist dadurch gekennzeichnet, dass $R^1$ für

$C_{1-3}$Alkyl steht und $R^2$ für Wasserstoff, Methyl oder Ethyl steht oder $R^1R^2N$ einen 5- bis 6gliedrigen Ring bedeutet.

Weitere bevorzugte Gruppen von erfindungsgemässen Verbindungen sind dadurch gekennzeichnet, dass Q für einen Thiophenring steht, dessen Einarbeitung in den Rest des Moleküls durch Bindungen in 2- und 5-Stellung erfolgt ist, und dass die Gruppe $XY(CH_2)_m$ für $CH_2-S-(CH_2)_{2-3}$ steht, oder dass Q für einen Thiophenring steht, dessen Einarbeitung in den Rest des Moleküls durch Bindungen in 2- und 4-Stellung erfolgt ist, und dass die Gruppe $XY(CH_2)_m$ für $CH_2-S-(CH_2)_{2-3}$ steht, oder dass Q für einen Benzolring steht, der durch Bindungen in 1- und 3-Stellung eingearbeitet ist, und dass die Gruppe $XY(CH_2)_m$ für $O(CH_2)_{3-4}$ steht.

In der allgemeinen Formel I bedeutet $R^1$ eine lineare $C_{1-6}$Alkylgruppe, vorzugsweise eine lineare $C_{1-3}$Alkylgruppe, und insbesondere eine Methyl- oder Ethylgruppe. $R^2$ steht für eine lineare $C_{1-6}$Alkylgruppe, vorzugsweise eine lineare $C_{1-3}$Alkylgruppe, und insbesondere eine Methyl- oder Ethylgruppe. Die Substituenten $R^1$und $R^2$ können unabhängig voneinander aus den angegebenen Gruppen ausgewählt werden. $R^1$ und $R^2$ können auch zusammen mit dem Stickstoffatom, an das sie gebunden sind, einen 5- oder 6gliedrigen Ring, beispielsweise einen Pyrrolidino- oder Piperidinoring, bilden, wobei ein Piperidinoring bevorzugt wird.

Alk bedeutet eine geradkettige Alkylenkette mit 1 bis 6 Kohlenstoffatomen, vorzugsweise 1 bis 3 Kohlenstoffatomen, und insbesondere eine Methylengruppe.

Q bedeutet einen Thiophen- oder Benzolring. Der Thiophenring kann durch Bindungen in 2- und 5-Stellung oder 2- und 4-Stellung in den Rest des Moleküls eingearbeitet sein. Verbindungen, bei denen der Thiophenring in 2- und 4-Stellung eingearbeitet ist, werden bevorzugt. Der Benzolring ist in den Rest des Moleküls durch Bindungen in 1- und 3-Stellung eingearbeitet.

Wenn Q einen Thiophenring bedeutet, dann steht X für die Methylengruppe und Y für ein Schwefelatom. In diesem Fall hat m den Wert 2 oder 3.

Wenn Q einen Benzolring bedeutet, dann steht X für ein Sauerstoffatom und Y für eine Einfachbindung. In diesem Fall hat m den Wert 3 oder 4.

$R^3$ bedeutet eine Aminogruppe oder die p-Aminophenylgruppe.

Die erfindungsgemässen Verbindungen werden durch ein Verfahren hergestellt, das dadurch gekennzeichnet ist, dass man eine Verbindung der allgemeinen Formel II:

$$R^1R^2NAlk-Q-XY(CH_2)_m-\overset{\overset{NH}{\|}}{C}-OCH_3 \qquad (II)$$

worin $R^1$, $R^2$, Alk, Q, X, Y und m die oben angegebenen Bedeutungen haben, in an sich bekannter Weise mit einem Sulfonamid III:

$$H_2N-\underset{\underset{O}{\|}}{\overset{\overset{O}{\|}}{S}}-R^3 \qquad (III)$$

worin $R^3$ die oben angegebene Bedeutung hat, umsetzt und die erhaltene Verbindung gegebenenfalls in ihr physiologisch annehmbares Salz umwandelt.

Bei diesem Verfahren wird eine Verbindung der allgemeinen Formel II mit einem Sulfonamid der allgemeinen Formel III zu den angestrebten Verbindungen umgesetzt. Die Umsetzung erfolgt in einem Lösungsmittel und bei einer Temperatur von Raumtemperatur bis Siedetemperatur des verwendeten Lösungsmittels. Geeignete Lösungsmittel sind z.B. Alkohole, wie Methanol oder Ethanol, Ether, wie Dioxan oder Tetrahydrofuran, und halogenierte Kohlenwasserstoffe, wie Tetrachlorkohlenstoff oder Chloroform. Die Aufarbeitung erfolgt in an sich bekannter Weise, beispielsweise duch Einengen des Reaktionsgemisches und chromatographische Abtrennung der erhaltenen Verbindung. Als Träger für die chromatographische Abtrennung können z.B. nichtionogene Adsorbenzien, wie Kieselgel etc., verwendet werden. Als Elutionsmittel dient beispielsweise ein Gemisch aus Alkohol und halogeniertem Kohlenwasserstoff, wie z.B. Methylenchlorid-Methanol, im Volumenverhältnis von 8:2.

Die erfindungsgemässen Verbindungen können mit geeigneten Säuren in ihre physiologisch annehmbaren Salze überführt werden. Diese Umsetzung erfolgt in bekannter Weise.

Geeignete Säuren sind anorganische und organische Säuren. Beispiele für anorganische Säuren sind Salzsäure und Bromwasserstoffsäure. Beispiele für geeignete organische Säuren sind Oxalsäure, Äpfelsäure und Bernsteinsäure. Grundsätzlich sind alle in der Pharmazie geeigneten anorganischen und organischen Säuren für die Umwandlung in das physiologisch annehmbare Salz einsetzbar.

Die erfindungsgemässen Verbindungen der allgemeinen Formeln I bilden leicht Säureadditionssalze, und es bestehen tautomere Formen dieser Verbindungen in der

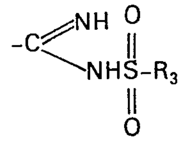 -Stellung.

Daher umfasst die vorliegende Erfindung auch die Herstellung der Säureadditionssalze und der tautomeren Formen der Verbindungen der allgemeinen Formeln I.

Die Verbindungen N-(p-Aminobenzolsulfonyl)-[(2-N,N-dimethylaminomethylthienyl-4)methylthio]propionamidin, N-(p-Aminobenzolsulfonyl)-3-[(2-piperidinylmethylthienyl-4)-methylthio]-propionamidin und N-(p-Aminobenzolsulfonyl)-4-[(3-piperidinylmethyl)phenoxy]butyroamidin sowie die physiologisch annehmbaren Säureadditionssalze werden bevorzugt. Ganz besonders bevorzugt werden N-(p-Aminobenzolsulfonyl)-3-[(2-N,N-dimethylaminomethylthienyl-4)methylthio]propionamidin, N-(p-Aminobenzolsulfonyl)-3-[(2-piperidinylmethylthienyl-4)methylthio]-propionamidin und die physiologisch annehmbaren Salze davon.

Die erfindungsgemässen Verbindungen, vorzugsweise in der Form eines Salzes, können zur Verabreichung in jeder beliebigen Weise formuliert werden. Die Erfindung umfasst daher auch Arzneimittel, die mindestens eine erfindungsgemässe Verbindung zur Verwendung in der Human- oder Veterinärmedizin enthalten. Solche Arzneimittel können herkömmlicherweise unter Verwendung von einem oder mehreren pharmazeutisch annehmbaren Träger oder Verdünnungsmittel hergestellt werden.

Die erfindungsgemässen Verbindungen können daher für die orale, bukkale, topische, parenterale oder rektale Verabreichung formuliert werden, wobei die orale Verabreichung bevorzugt wird. Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind. Für die bukkale Verabreichung kann das Arzneimittel die Form von Tabletten oder Briefchen einnehmen, die in herkömmlicher Weise formuliert worden sind.

Die erfindungsgemässen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen. Die Arzneimittel können solche Formen, wie Suspensionen, Lösungen oder Emulsionen in öligen oder wässrigen Trägern, einnehmen und sie können Formulierungshilfsmittel, wie Suspendierungs-, Stabilisierungs- und/oder Dispergierungsmittel, enthalten. Alternativ kann der Wirkstoff auch in Pulverform zur Rekonstitution mit einem geeigneten Träger, z.B. sterilem, pyrogenfreien Wasser, vor dem Gebrauch vorliegen.

Die erfindungsgemässen Verbindungen können auch für rektale Zubereitungen, z.B. Suppositorien oder Retentionseinläufe, formuliert werden, die z.B. herkömmliche Suppositoriengrundlagen, wie Kakaobutter oder andere Glyceride, enthalten.

Zur topischen Anwendung können die erfindungsgemässen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemässen Verbindungen 1 bis 4 Dosen bis insgesamt 5 mg bis 1 g/Tag, vorzugsweise 5 bis 250 mg/Tag, je nach Zustand des Patienten. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es z.B. Fälle, wo mit weniger als der oben genannten Mindestmenge ausgekommen werden kann, während in anderen Fällen die genannte obere Grenze überschritten werden muss.

Die erfindungsgemässen Verbindungen zeichnen sich gegenüber anerkannt guten Arzneimitteln der gleichen Wirkungsrichtung durch eine Verbesserung der pharmakologischen Aktivitäten aus. Dies ergibt sich aus den Ergebnissen der im folgenden dargestellten pharmakologischen Vergleichsuntersuchungen.

Eine anerkannte Methode zur Bestimmung $H_2$-antagonistischer Wirksamkeit ist die Ermittlung der $pA_2$-Werte in vitro am isolierten Meerschweinchenvorhof (vgl. Ariens, Molecular Pharmacology, Bd. 1, Academic Press, New York, 1964).

| | $pA_2$-Werte |
|---|---|
| Cimetidin: | 6,3 (Vergleich) |
| Beispiel 2: | 7,10 |
| Beispiel 8: | 6,95 |
| Beispiel 10: | 7,20 |

Andere Verbindungen der allgemeinen Formel I zeigen ähnliche pharmakologische Wirkungen.

Beispiel 1

N-Sulfamoyl-3-[(2-N,N-dimethylaminomethylthienyl-4)methylthio]propionamidin

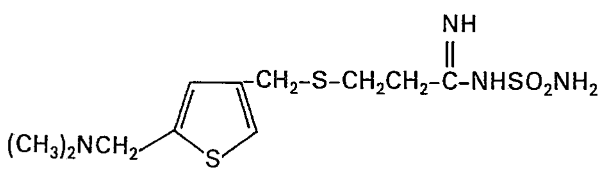

Zu einer Lösung aus 2,56 g (10 mmol) Methyl-3-[(2-N,N-dimethylaminomethylthienyl-4)methylthio]propionimidat und 30 ml Methanol gibt man 2,40 g (25 mmol) Sulfamid und lässt 8 h unter Rückfluss reagieren. Anschliessend wird das Lösungsmittel im Vakuum abdestilliert und der Rückstand säulenchromatographisch (Kieselgel; Elutionsmittel: Methylenchlorid/Methanol 80:20) gereinigt.

Farbloses Öl. $R_f = 0{,}2$ ($CH_2Cl_2$/MeOH 90:10)
Ausbeute: 1,51 g (45%)

$C_{11}H_{20}N_4S_3O_2$ (336)

| | C | H | N |
|---|---|---|---|
| Ber.: | 39,28 | 5,95 | 16,67 |
| Gef.: | 38,94 | 6,12 | 16,71 |

$^1$H-NMR-Spektrum: ($d_6$-DMSO, TMS als interner Standard):

$\delta$ = 2,13 (s) ($(CH_3)_2$-N) 6H,
2,27–2,77 (m) ($-(CH_2)_2-$) 4H,
3,53 (s) (S–$CH_2$) 2H,
3,67 (s) (N–$CH_2$) 2H,
6,43 (s) ($-SO_2NH_2$) 2H (austauschbar mit $D_2O$),
6,87 (s) (Aromaten-H) 1H,
7,20 (s) (Aromaten-H) 1H,
7,30 (s) (NH–$SO_2$) 1H (austauschbar mit $D_2O$),
8,20 (s) (=N–H) 1H (austauschbar mit $D_2O$) ppm

Beispiel 1a

Herstellung von Methyl-3-[(2-N,N-dimethylaminomethylthienyl-4)methylthio]propionimidat

Die Herstellung von Methyl-3-[(2-N,N-dimethylaminomethylthienyl-4)methylthio]propionimidat erfolgt durch Umsetzung von 2-(N,N-Dimethylaminomethyl)-4-S-isothioharnstoff-methylthiophen-dihydrochlorid mit äquimolaren Mengen Chlorpropionitril in wässriger, alkalischer Lösung bei 0–10°C.

Anschliessend wird das gewonnene [(2-(N,N-Dimethylaminomethyl)-4-thienylmethylthio]propionitril in wasserfreiem Methanol mit trockenem Chlorwasserstoff bei 0°C zum Methyl-3-[(2-N,N-dimethylaminomethylthienyl-4)methylthio]propionimidat umgesetzt.

Beispiel 2

N-(p-Aminobenzolsulfonyl)-3-[(2-N,N-dimethylaminomethylthienyl-4)methylthio]propionamidin

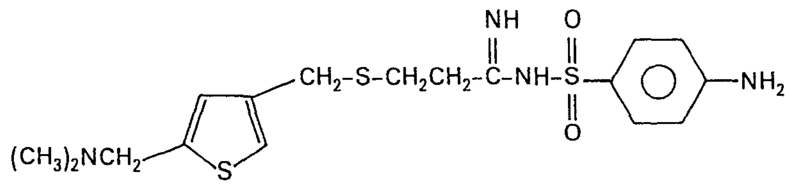

Die Reaktion erfolgt analog Beispiel 1 mit 2,72 g (10 mmol) Methyl-3-[(2-N,N-dimethylaminomethylthienyl-4)methylthio]-propionimidat und 4,3 g (25 mmol) p-Aminobenzolsulfonamid.

Farbloses Öl. $R_f = 0,4$ ($CH_2Cl_2$/MeOH 80:20)
Ausbeute: 2,3 g (56%)

$C_{17}H_{24}N_4O_2S_3$ (412)

Ber.: C 49,51; H 5,83; N 13,59
Gef.: C 49,35; H 5,78; N 13,31

$^1$H-NMR-Spektrum ($d_6$-DMSO, TMS als interner Standard):

δ = 2,17 (s) ($N-CH_3)_2$) 6H,
2,40–2,67 (m) ($-(CH_2)_2-$4H,
3,53 (s) ($S-CH_2$) 2H,
3,63 (s) ($N-CH_2$) 2H,
5,77 (s) ($-NH_2$) 2H (austauschbar mit $D_2O$),
6,53 (d) (Aromaten-H) 2H,
6,83 (s) (Aromaten-H) 1H,
7,13 (s) (Aromaten-H) 1H,
7,43 (d) (Aromaten-H) 2H,
7,73 (s) ($=NH-SO_2$) 1H (austauschbar mit $D_2O$),
8,40 (s) ($=NH$) 1H (austauschbar mit $D_2O$) ppm

Beispiel 3

N-Sulfamoyl-4-[(3-N,N-dimethylaminomethyl)-phenoxy]butyroamidin

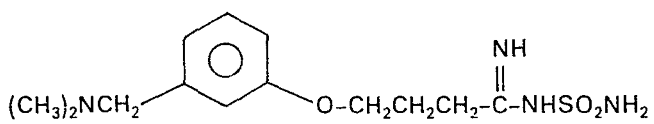

Die Reaktion erfolgt analog Beispiel 1 mit 2,50 g (10 mmol) Methyl-4-[(3-N,N-dimethylaminomethyl)phenoxy]butyroimidat und 2,40 g (25 mmol) Sulfamid.

Hellgelbes Öl. $R_f = 0,2$ ($CH_2Cl_2$/MeOH 50:50)
Ausbeute: 0,88 g (28%)

$C_{13}H_{22}N_4O_3S$

Ber.: C 49,68 H 7,01 N 17,83
Gef.: C 49,37 H 6,82 N 17,20

$^1$H-NMR-Spektrum ($d_6$-DMSO, TMS als interner Standard):

δ = 1,80–2,53 (m) ($-CH_2-CH_2-$) 4H,
2,17 (s) ($(CH_3)_2N-$) 6H,
3,40 (s) ($N-CH_2$) 2H,
4,00 (t) ($-OCH_2$) 2H,
6,50 (s) ($-NH_2$) 2H (austauschbar mit $D_2O$),
6,67–7,40 (m) (Aromaten-H) 4H,
7,40 (s) ($NHSO_2-$) 1H (austauschbar mit $D_2O$),
8,30 (s) ($=N-H$) 1H (austauschbar mit $D_2O$) ppm

Beispiel 3a

Herstellung von Methyl-4-[(3-N,N-dimethylaminomethyl)-phenoxy]butyroimidat

Die Herstellung von Methyl-4-[(3-N,N-dimethylaminomethyl)-phenoxy]butyroimidat gelingt durch Umsetzung von 3-(Dimethylaminomethyl)-phenol in wasserfreiem Dimethylformamid mit NaH und 4-Chlorbuttersäurenitril zum 3-N,N-Dimethylaminomethylphenoxybutyronitril, das anschliessend in wasserfreiem Methanol-Chloroform-Gemisch bei 0–10 °C mit trockenem Chlorwasserstoff zum Methyl-4-[(3-N,N-dimethylaminomethyl)-phenoxy]butyroimidat reagiert.

Beispiel 4

N-(p-Aminobenzolsulfonyl)-4-[(3-N,N-dimethylaminomethyl)-phenoxy]butyroamidin

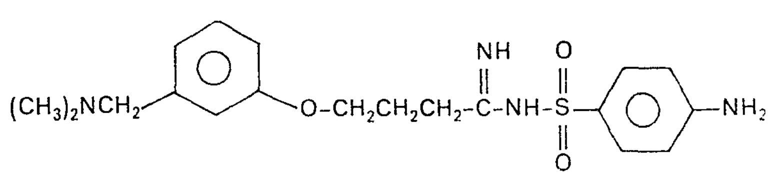

Die Reaktion erfolgt analog Beispiel 1 mit 2,50 g (10 mmol) Methyl-4-[(3-N,N-dimethylaminomethyl)-phenoxy]butyroimidat und 4,3 g (25 mmol) p-Aminobenzolsulfonamid.

Farbloses Öl. $R_f = 0{,}4$ ($CH_2Cl_2$/MeOH 50:50)
Ausbeute: 1,48 g (38%)

$C_{19}H_{26}N_4O_3S$

Ber.: C 58,44 H 6,71 N 14,34
Gef.: C 58,14 H 6,74 N 14,06

$^1$H-NMR-Spektrum ($d_6$-DMSO, TMS als interner Standard)

$\delta$ = 1,87 (m) ($-CH_2-$) 2H,
2,13 (s) ($N-(CH_3)_2$) 6H,
2,37 (t) ($C-CH_2$) 2H,
3,37 (s) ($N-CH_2$) 2H,
3,87 (t) ($-O-CH_2$) 2H,
5,80 (s) ($-NH_2$) 2H (austauschbar mit $D_2O$),
6,60 (d) (Aromaten-H) 2H,
6,73–7,33 (m) (Aromaten-H) 4H,
7,50 (d) (Aromaten-H) 2H,
7,77 (s) ($-NH-SO_2$) 1H (austauschbar mit $D_2O$),
8,47 (s) ($=NH$) 1H (austauschbar mit $D_2O$) ppm

Beispiel 5

N-Sulfamoyl-4-[(3-piperidinylmethyl)-phenoxy]butyroamidin

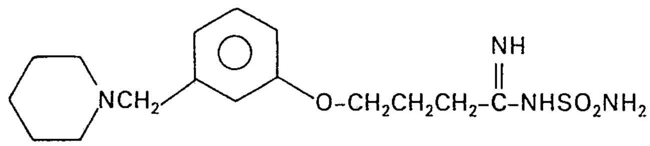

Die Reaktion erfolgt analog Beispiel 1 mit 2,90 g (10 mmol) Methyl-4-[(3-piperidinylmethyl)-phenoxy]butyroimidat und 2,40 g (25 mmol) Sulfamid.

Farbloses Öl. $R_f = 0{,}3$ ($CH_2Cl_2$/MeOH 80:20)
Ausbeute: 1,06 g (30%)
$C_{16}H_{26}N_4O_3S$ (354)
$^1$H-NMR-Spektrum ($d_6$-DMSO, TMS als interner Standard):

$\delta$ = 1,43 (s, breit) ($-(CH_2)_3-$) 6H,
2,0 (m) ($-CH_2-CH_2-CH_2$) 2H,
2,33 (s, breit) ($N(CH_2)_2-;-CH_2$) 6H,
3,43 (s) ($N-CH_2$) 2H,
4,00 (t) ($-O-CH_2$) 2H,
6,47 (s) ($-SO_2NH_2$) 2H (austauschbar mit $D_2O$),
6,67–7,30 (m) (Aromaten-H) 4H,
7,33 (s) ($NH-SO_2$) 1H (austauschbar mit $D_2O$),
8,23 (s) ($=N-H$) 1H (austauschbar mit $D_2O$) ppm

Die Herstellung des Methyl-4-[(3-piperidinylmethyl)-phenoxy]butyroimidats erfolgt analog Beispiel 5a aus 3-(Piperidinylmethyl)-phenol und 4-Chlorbuttersäurenitril und anschliessender Reaktion mit Chlorwasserstoff.

Beispiel 6

N-(p-Aminobenzolsulfonyl)-4-[(3-piperidinylmethyl)phenoxy]-butyroamidin

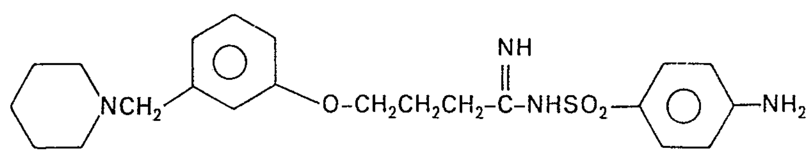

Die Reaktion erfolgt analog Beispiel 1 mit 2,90 g (10 mmol) Methyl-4-[(3-piperidinylmethyl)-phenoxy]butyroimidat und 4,30 g (25 mmol) p-Aminobenzolsulfonamid.

Hellgelbes Öl. $R_f = 0{,}3$ ($CH_2Cl_2$/MeOH 80:20)
Ausbeute: 1,5 g (35%)

$C_{22}H_{30}N_4O_3S$ (430)

Ber.: C 61,39 H 6,98 N 13,02
Gef.: C 61,44 H 6,96 N 12,84

$^1$H-NMR-Spektrum ($d_6$-DMSO, TMS als interner Standard):

$\delta$ = 1,43 (s, breit) ($-(CH_2)_3-$) 6H,
1,90 (m) ($-CH_2-CH_2-CH_2-$) 2H,
2,33 (s, breit) ($N(CH_2)_2;-CH_2$) 6H,
3,37 (s) ($N-CH_2$) 2H,
3,87 (t) ($-O-CH_2$) 2H,
5,80 (s) ($-NH_2$) 2H (austauschbar mit $D_2O$),
6,60 (d) (Aromaten-H) 2H,
6,73–7,33 (m) (Aromaten-H) 4H,
7,50 (D) (Aromaten-H) 2H,
7,77 (s) ($NHSO_2$) 1H (austauschbar mit $D_2O$),
8,43 (s)($=N-H$) 1H (austauschbar mit $D_2O$) ppm

Beispiel 7

N-Sulfamoyl-5-[(3-piperidinylmethyl)-phenoxy]valeroamidin

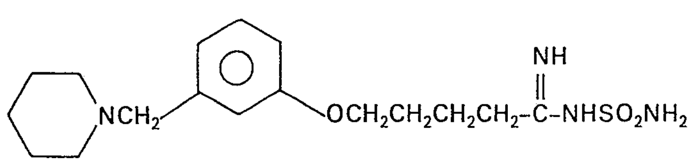

Die Reaktion erfolgt analog Beispiel 1 mit 3,04 g (10 mmol) Methyl-5-[(3-piperidinylmethyl)-phenoxy]valeroimidat und 2,40 g (25 mmol) Sulfamid.

Gelbes Öl. $R_f$ = 0,3 ($CH_2Cl_2/CH_3OH$ 80:20)
Ausbeute: 1,18 g (32%)

$C_{17}H_{28}N_4O_3S$ (369)

Ber.: C 55,41 H 7,66 N 15,20
Gef.: C 55,00 H 7,53 N 15,60

$^1$H-NMR-Spektrum ($d_6$-DMSO, TMS als interner Standard)

$\delta$ = 1,43 (s, breit) ($-(CH_2)_3-$) ($-CH_2-$) 8H,
1,73 (m) ($-CH_2-CH_2-CH_2$) 2H,
2,33 (s, breit) ($N(CH_2)_2$;$CH_2-C{=}NH$) 6H,
3,37 (s) ($N-CH_2$) 2H,
3,97 (t) ($-O-CH_2$) 2H,
6,43 (s) ($SO_2-NH_2$) 2H (austauschbar mit $D_2O$)
6,70–7,33 (m) (Aromaten-H), 4H,
7,37 (s) ($NHSO_2$) 1H (austauschbar mit $D_2O$)
8,20 (s) ($=N-H$) 1H (austauschbar mit $D_2O$) ppm

Die Herstellung des Methyl-5-[(3-piperidinylmethyl)-phenoxy]valeroimidats erfolgt analog Beispiel 3a aus 3-(Piperidinylmethyl)-phenol und 5-Chlorvaleriansäurenitril und anschliessender Reaktion mit Chlorwasserstoff.

Beispiel 8

N-(p-Aminobenzolsulfonyl)-5-[(3-piperidinylmethyl)phenoxy]-valeroamidin

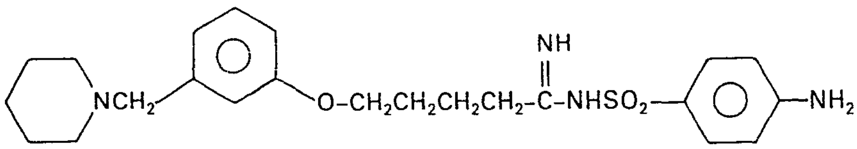

Die Reaktion erfolgt analog Beispiel 1 mit 3,04 g (10 mmol) Methyl-5-[(3-piperidinylmethyl)-phenoxy]valeroimidat und 4,30 g (25 mmol) p-Aminobenzolsulfonamid.

Hellgelbes Öl. $R_f$ = 0,3 ($CH_2Cl_2$/MeOH 80:20)
Ausbeute: 1,69 g (38%)

$C_{23}H_{32}N_4O_3S$ (445)

Ber.: C 62,14 H 7,26 N 12,60
Gef.: C 62,30 H 7,17 N 12,65

$^1$H-NMR-Spektrum ($d_6$-DMSO, TMS als interner Standard),

$\delta$ = 1,43 (s, breit) ($-(CH_2)_3-$;$-CH_2$) 8H,
1,63 (m) ($-CH_2-CH_2-CH_2$) 2H,
2,30 (s, breit) ($N(CH_2)_2$;$CH_2-C{=}NH$) 6H,
3,37 (s) ($N-CH_2$) 2H,
3,87 (t) ($O-CH_2$) 2H,
5,80 (s) ($-NH_2$) 2H (austauschbar mit $D_2O$),
6,60 (d) (Aromaten-H) 2H,
6,70–7,33 (m) (Aromaten-H) 4H,
7,53 (d) (Aromaten-H) 2H,
7,73 (s) ($NH-SO_2$) 1H (austauschbar mit $D_2O$),
8,37 (s) ($=N-H$) 1H (austauschbar mit $D_2O$) ppm

Beispiel 9

N-Sulfamoyl-3-[(2-piperidinylmethylthienyl-4)methylthio]propionamidin

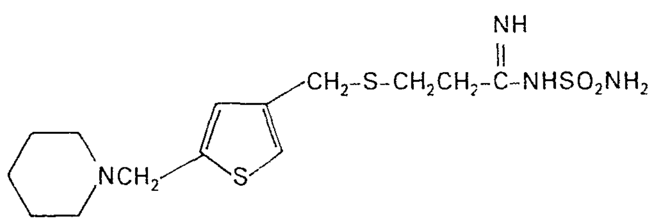

Die Reaktion erfolgt analog Beispiel 1 aus 3,12 g (10 mmol) Methyl-3-[(2-piperidinylmethylthienyl-4)methylthio]propionimidat und 2,40 g (25 mmol) Sulfamid.

Farbloses Öl. $R_f$ = 0,2 ($CH_2Cl_2$/MeOH 80:20)
Ausbeute: 0,79 g (21%)

$^1$H-NMR-Spektrum ($d_6$-DMSO, TMS als interner Standard),

$\delta$ = 1,47 (m, breit) ($-(CH_2)_3-$) 6H,
2,13–2,77 (m) ($-(CH_2)_2-$,$N-(CH_2)_2-$) 8H,
3,70 (s, breit) ($-CH_2-S$, $N-CH_2$) 4H,
6,43 (s) ($-SO_2NH_2$) 2H (austauschbar mit $D_2O$),
6,80 (s) ($NH-SO_2$) 1H (austauschbar mit $D_2O$),
6,93 (s) (Aromaten-H) 1H,
7,23 (s) (Aromaten-H) 1H,
7,30 (s) ($=N-H$)) 1H (austauschbar mit $D_2O$) ppm

Die Herstellung von Methyl-3-[(2-piperidinylmethylthienyl-4)methylthio]proprionimidat erfolgt analog dem Herstellungsverfahren aus 2-(Piperidinylmethyl)-4-S-isothioharnstoffmethylthiophen-dihydrochlorid, äquimolaren Mengen Chlorpropionitril und anschliessender Umsetzung mit Methanol-Chlorwasserstoff, wie unter Beispiel 1a beschrieben.

Beispiel 10

N-(p-Aminobenzolsulfonyl)-3-[(2-piperidinylmethylthienyl-4)methylthio]propionamidin

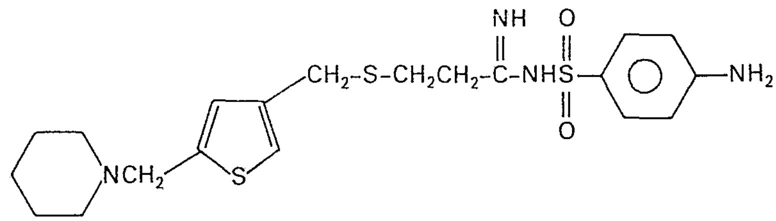

Die Reaktion erfolgt analog Beispiel 1 aus 3,12 g (10 mmol) Methyl-3-[(2-piperidinylmethylthienyl-4)methylthio]propionimidat und 4,3 g (25 mmol) p-Aminobenzolsulfonamid.

Hellgelbes Öl. $R_f = 0,2$ ($CH_2Cl_2$/MeOH 90:10)
Ausbeute: 1,85 g (41%)

$C_{20}H_{28}N_4O_2S_3$ (452)

Ber.: C 53,10 H 6,19
Gef.: C 53,42 H 6,39

$^1$H-NMR-Spektrum ($d_6$-DMSO, TMS als interner Standard),

$\delta$ = 1,47 (m, breit) ($-(CH_2)_3-$) 6H,
2,30–2,73 (m) ($-(CH_2)_2-$,$N-(CH_2)_2-$) 8H,
3,47 (s) ($CH_2$-S) 2H,
3,70 (s) (N-$CH_2$) 2H,
5,83 (s) ($-NH_2$) 2H (austauschbar mit $D_2O$)
6,63 (d) (Aromaten-H) 2H,
6,90 (s) (Aromaten-H) 1H,
7,20 (s) (Aromaten-H), 1H,
7,50 (d) (Aromaten-H) 2H,
7,80 (s) (NH-$SO_2$) 1H (austauschbar mit $D_2O$),
8,47 (s) (=N-H) 1H (austauschbar mit $D_2O$) ppm

Nachstehend werden Beispiele für erfindungsgemässe Arzneimittel angegeben:

a)

| Tabletten | mg/Tablette | mg/Tablette |
|---|---|---|
| Wirkstoff | 20,0 | 40,0 |
| mikrokristalline Cellulose BPC | 99,5 | 199,0 |
| Magnesiumstearat B.P. | 0,5 | 1,0 |
| Kompressionsgewicht | 120,0 | 240,0 |

Das Arzneimittel wird durch ein 250-µm-Sieb gesiebt, mit den Streckmitteln vermischt und unter Verwendung von Stempeln mit einem Durchmesser von 6,5 mm bzw. 8,0 mm für die 20 und 40 mg Stärke komprimiert. Die Tabletten können mit geeigneten filmbildenden Materialien, z.B. Methylcellulose, Äthylcellulose oder Hydroxypropylmethylcellulose, nach Standardtechniken filmbeschichtet werden.

b)

| Kapseln | mg/Kapsel |
|---|---|
| Wirkstoff | 20,0 |
| Sta-R×1500 Stärke | 79,5 |
| Magnesiumstearat B.P. | 0,5 |
| Füllgewicht | 100,0 |

Der Wirkstoff wird durch ein 250-µm-Sieb gesiebt und mit anderen Materialien vermengt. Das Gemisch wird unter Verwendung einer geeigneten Abfüllmaschine in Hartgelatinekapseln Nr. 3 abgefüllt. Andere Dosen können hergestellt werden, indem man das Füllgewicht erhöht und erforderlichenfalls die Kapselgrösse entsprechend verändert.

c)

| Tabletten mit verzögerter Freisetzung | mg/Tablette |
|---|---|
| Wirkstoff | 80 |
| Cutina HR* | 25 |
| Lactose B.P. | 142,5 |
| Magnesiumstearat B.P. | 2,5 |
| Kompressionsgewicht | 250,0 |

* Cutina HR ist eine Sorte von mikrofeinem, hydriertem Rizinusöl von Sipon Products Ltd., London.

Das Arzneimittel wird durch ein 250-µm-Sieb gesiebt und mit dem Cutina HR und der Lactose vermengt. Das gemischte Pulver wird mit technischem Methylsprit 74 O.P. befeuchtet und granuliert. Das Granulat wird getrocknet, gesiebt und mit dem Magnesiumstearat vermengt. Die geschmierten Körner werden unter Verwendung von 8,5 mm Stempeln zu Tabletten mit einer Härte von nicht mehr als 10 kp (schleuniger Tester) verpresst.

d) Injizierbare Zubereitung für die intravenöse Verabreichung

| | %Gew./Vol. |
|---|---|
| Wirkstoff | 0,25 |
| Wasser zur Injektion BP auf | 100,00 |

Natriumchlorid kann zugegeben werden, um die Tonizität der Lösung einzustellen. Der

pH-Wert kann mit verdünnter Säure oder Alkali so eingestellt werden, dass die maximale Stabilität erhalten wird.

Die Lösung wird hergestellt, geklärt und unter Stickstoff in Ampullen mit geeigneter Grösse eingefüllt, die geschmolzen werden. Die injizierbare Zubereitung wird durch Erhitzen in einem Autoklaven unter Verwendung eines annehmbaren Zyklus sterilisiert. Alternativ kann die Lösung durch Filtration sterilisiert und bei aseptischen Bedingungen in sterile Ampullen abgefüllt werden.

e)

| Sirup | mg/5 ml Dosis |
|---|---|
| Wirkstoff | 20,0 |
| Saccharose | 2750,0 |
| Glycerin | 500,0 |
| Puffer | wie erforderlich |
| Aromastoff | wie erforderlich |
| Färbemittel | wie erforderlich |
| Konservierungsmittel | wie erforderlich |
| destilliertes Wasser | auf 5,0 ml |

Der Wirkstoff, der Puffer, der Aromastoff, das Konservierungsmittel und das Färbemittel werden in einem Teil des Wassers aufgelöst. Der Rest des Wassers wird auf etwa 80°C erhitzt, und die Saccharose wird darin aufgelöst. Das Gemisch wird abgekühlt. Die zwei Lösungen werden vermischt, auf das Volumen eingestellt und durch Filtration geklärt.

**Patentansprüche für die Vertragsstaaten BE, CH, DE, FR, GB, IT, LI, LU, NL, SE**

1. Heterocyclische Verbindungen der allgemeinen tautomeren Formeln I:

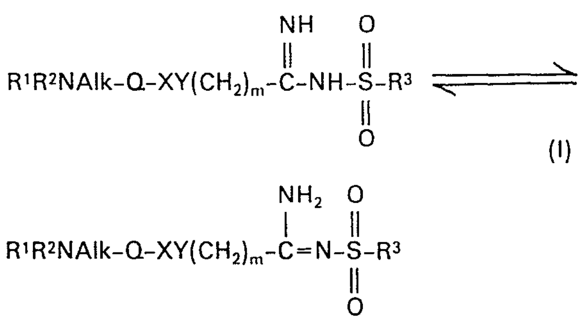

(I)

in denen $R^1$ für lineares $C_{1-6}$Alkyl und $R^2$ für lineares $C_{1-6}$Alkyl steht oder wobei $R^1$ und $R^2$ zusammen mit dem Stickstoffatom, an das sie gebunden sind, einen 5- oder 6gliedrigen Ring bilden;

Alk für eine geradkettige Alkylenkette mit 1 bis 6 Kohlenstoffatomen steht;

Q für einen Thiophenring steht, dessen Einarbeitung in den Rest des Moleküls durch Bindungen in 2- und 5-Stellung oder in 2- und 4-Stellung erfolgt ist, oder wobei Q für einen Benzolring steht, dessen Einarbeitung in den Rest des Moleküls durch Bindungen in 1- und 3-Stellung erfolgt ist; X in dem Fall, dass Q die Bedeutung Thiophen hat, für Methylen, Y für Schwefel steht und m den Wert 2 oder 3 hat;

X in dem Fall, dass Q die Bedeutung Benzol hat, für Sauerstoff, Y für eine Einfachbindung steht und m den Wert 3 oder 4 hat;

$R^3$ für eine Aminogruppe oder eine p-Aminophenylgruppe steht;

sowie die physiologisch annehmbaren Salze davon.

2. Verbindungen nach Anspruch 1, dadurch gekennzeichnet, dass $R^1$ für $C_{1-3}$Alkyl steht und $R^2$ für Wasserstoff, Methyl oder Ethyl steht oder $R^1R^2N$ einen 5- bis 6gliedrigen Ring bedeutet.

3. Verbindungen nach Anspruch 1 oder 2, dadurch gekennzeichnet, dass die Gruppe Alk für Methylen steht.

4. Verbindungen nach einem der Ansprüche 1 bis 3, dadurch gekennzeichnet, dass Q für einen Thiophenring steht, dessen Einarbeitung in den Rest des Moleküls durch Bindungen in 2- und 5-Stellung erfolgt ist, und dass die Gruppe $XY(CH_2)_m$ für $CH_2$-S-$(CH_2)_{2-3}$ steht.

5. Verbindungen nach einem der Ansprüche 1 bis 3, dadurch gekennzeichnet, dass Q für einen Thiophenring steht, dessen Einarbeitung in den Rest des Moleküls durch Bindungen in 2- und 4-Stellung erfolgt ist, und dass die Gruppe $XY(CH_2)_m$ für $CH_2$-S-$(CH_2)_{2-3}$ steht.

6. Verbindungen nach einem der Ansprüche 1 bis 3, dadurch gekennzeichnet, dass Q für einen Benzolring steht, der durch Bindungen in 1- und 3-Stellung eingearbeitet ist, und dass die Gruppe $XY(CH_2)_m$ für $O(CH_2)_{3-4}$ steht.

7. N-(p-Aminobenzolsulfonyl)-3-[(2-N,N-dimethylaminomethylthienyl-4)methylthio]propionamidin und die physiologisch annehmbaren Salze davon.

8. N-(p-Aminobenzolsulfonyl)-4-[(3-N,N-dimethylaminomethyl)phenoxy]butyroamidin und die physiologisch annehmbaren Salze davon.

9. N-(p-Aminobenzolsulfonyl)-4-[3-(1-piperidinylmethyl)phenoxy]butyroamidin und die physiologisch annehmbaren Salze davon.

10. N-Sulfamoyl-3-[(2-N,N-dimethylaminomethylthienyl-4)methylthio]propionamidin und die physiologisch annehmbaren Salze davon.

11. N-Sulfamoyl-4-[(3-N,N-dimethylaminomethyl)phenoxy]-butyroamidin und die physiologisch annehmbaren Salze davon.

12. N-Sulfamoyl-4-[(3-piperidinylmethyl)phenoxy]butyroamidin und die physiologisch annehmbaren Salze davon.

13. N-Sulfamoyl-5-[(3-piperidinylmethyl)phenoxy]valeroamidin und die physiologisch annehmbaren Salze davon.

14. N-(p-Aminobenzolsulfonyl)-5-[(3-piperidinylmethyl)phenoxy]valeroamidin und die physiologisch annehmbaren Salze davon.

15. N-Sulfamoyl-3-[(2-(1-piperidinylmethyl) thienyl-4)-methylthio]propionamidin und die physiologisch annehmbaren Salze davon.

16. N-(p-Aminobenzolsulfonyl)-3-[(2-(1-piperidinylmethyl)thienyl-4)methylthio]propionamidin

und die physiologisch annehmbaren Salze davon.

17. N-Sulfamoyl-[(2-piperidinylmethylthienyl-4)methylthio]propionamidin und die physiologisch annehmbaren Salze davon.

18. N-(p-Aminobenzolsulfonyl)-3-[(2-piperidinylmethylthienyl-4)methylthio]propionamidin und die physiologisch annehmbaren Salze davon.

19. N-(p-Aminobenzolsulfonyl)-3-[(2-piperidinylmethylthiazol-4-yl)methylthio]propionamidin und die physiologisch annehmbaren Salze davon.

20. Verfahren zur Herstellung von Verbindungen nach Anspruch 1, dadurch gekennzeichnet, dass man eine Verbindung der allgemeinen Formel II:

$$R^1R^2NAlk\text{-}Q\text{-}XY(CH_2)_m\text{-}\overset{\overset{NH}{\|}}{C}\text{-}OCH_3 \qquad (II)$$

worin $R^1$, $R^2$, Alk, Q, X, Y und m die in Anspruch 1 angegebenen Bedeutungen haben, in an sich bekannter Weise mit einem Sulfonamid III:

$$H_2N\text{-}\underset{\underset{O}{\|}}{\overset{\overset{O}{\|}}{S}}\text{-}R^3 \qquad (III)$$

worin $R^3$ die in Anspruch 1 angegebene Bedeutung hat, umsetzt und die erhaltene Verbindung gegebenenfalls in ihr physiologisch annehmbares Salz umwandelt.

21. Arzneimittel, dadurch gekennzeichnet, dass es eine Verbindung nach Anspruch 1 und mindestens einen inerten, pharmazeutisch annehmbaren Träger oder ein inertes, pharmazeutisch annehmbares Verdünnungsmittel, gegebenenfalls zusammen mit mindestens einem weiteren Wirkstoff, enthält.

**Patentansprüche für den Vertragsstaat AT**

1. Verfahren zur Herstellung von heterocyclischen Verbindungen der allgemeinen tautomeren Formeln I:

$$R^1R^2NAlk\text{-}Q\text{-}XY(CH_2)_m\text{-}\overset{\overset{NH}{\|}}{C}\text{-}NH\text{-}\underset{\underset{O}{\|}}{\overset{\overset{O}{\|}}{S}}\text{-}R^3 \rightleftharpoons \qquad (I)$$

$$R^1R^2NAlk\text{-}Q\text{-}XY(CH_2)_m\text{-}\overset{\overset{NH_2}{|}}{C}{=}N\text{-}\underset{\underset{O}{\|}}{\overset{\overset{O}{\|}}{S}}\text{-}R^3$$

in denen $R^1$ für lineares $C_{1-6}$Alkyl und $R^2$ für lineares $C_{1-6}$Alkyl steht oder wobei $R^1$ und $R^2$ zusammen mit dem Stickstoffatom, an das sie gebunden sind, einen 5- oder 6gliedrigen Ring bilden;

Alk für eine geradkettige Alkylenkette mit 1 bis 6 Kohlenstoffatomen steht;

Q für einen Thiophenring steht, dessen Einarbeitung in den Rest des Moleküls durch Bindungen in 2- und 5-Stellung oder in 2- und 4-Stellung erfolgt ist, oder wobei Q für einen Benzolring steht, dessen Einarbeitung in den Rest des Moleküls durch Bindungen in 1- und 3-Stellung erfolgt ist;

X in dem Fall, dass Q die Bedeutung Thiophen hat, für Methylen, Y für Schwefel steht und m den Wert 2 oder 3 hat; X in dem Fall, dass Q die Bedeutung Benzol hat, für Sauerstoff, Y für eine Einfachbindung steht und m den Wert 3 oder 4 hat; $R^3$ für eine Aminogruppe oder eine p-Aminophenylgruppe steht;

sowie der physiologisch annehmbaren Salze davon, dadurch gekennzeichnet, dass man eine Verbindung der allgemeinen Formel II:

$$R^1R^2NAlk\text{-}Q\text{-}XY(CH_2)_m\text{-}\overset{\overset{NH}{\|}}{C}\text{-}OCH_3 \qquad (II)$$

worin $R^1$, $R^2$, Alk, Q, X, Y und m die oben angegebenen Bedeutungen haben, in an sich bekannter Weise mit einem Sulfonamid III:

$$H_2N\text{-}\underset{\underset{O}{\|}}{\overset{\overset{O}{\|}}{S}}\text{-}R^3 \qquad (III)$$

worin $R^3$ die oben angegebene Bedeutung hat, umsetzt und die erhaltene Verbindung gegebenenfalls in ihr physiologisch annehmbares Salz umwandelt.

2. Verfahren nach Anspruch 1, dadurch gekennzeichnet, dass $R^1$ für $C_{1-3}$Alkyl steht und $R^2$ für Wasserstoff, Methyl oder Ethyl steht, oder $R^1R^2N$ einen 5- bis 6gliedrigen Ring bedeutet.

3. Verfahren nach Anspruch 1 oder 2, dadurch gekennzeichnet, dass die Gruppe Alk für Methylen steht.

4. Verfahren nach einem der Ansprüche 1 bis 3, dadurch gekennzeichnet, dass Q für einen Thiophenring steht, dessen Einarbeitung in den Rest des Moleküls durch Bindungen in 2- und 5-Stellung erfolgt ist, und dass die Gruppe $XY(CH_2)_m$ für $CH_2\text{-}S\text{-}(CH_2)_{2-3}$ steht.

5. Verfahren nach einem der Ansprüche 1 bis 3, dadurch gekennzeichnet, dass Q für einen Thiophen- oder Thiazolring steht, dessen Einarbeitung in den Rest des Moleküls durch Bindungen in 2- und 4-Stellung erfolgt ist, und dass die Gruppe $XY(CH_2)_m$ für $CH_2\text{-}S\text{-}(CH_2)_{2-3}$ steht.

6. Verfahren nach einem der Ansprüche 1 bis 3, dadurch gekennzeichnet, dass Q für einen Benzolring steht, der durch Bindungen in 1- und 3-Stellung eingearbeitet ist, und dass die Gruppe $XY(CH_2)_m$ für $O(CH_2)_{3-4}$ steht.

7. Verfahren nach Anspruch 1, dadurch gekennzeichnet, dass man N-(p-Aminobenzolsulfonyl)-3-[(2-N,N-dimethylaminomethylthienyl-4)methylthio]propionamidin und die physiologisch annehmbaren Salze davon herstellt.

8. Verfahren nach Anspruch 1, dadurch gekennzeichnet, dass man N-(p-Aminobenzolsulfonyl)-4-[(3-N,N-dimethylaminomethyl)-phenoxy]butyroamidin und die physiologisch annehmbaren Salze davon herstellt.

9. Verfahren nach Anspruch 1, dadurch gekennzeichnet, dass man N-(p-Aminobenzolsulfonyl)-4-[3-(1-piperidinylmethyl)-phenoxy]butyroamidin und die physiologisch annehmbaren Salze davon herstellt.

10. Verfahren nach Anspruch 1, dadurch gekennzeichnet, dass man N-Sulfamoyl-3-[(2-N,N-dimethylaminomethylthienyl-4)methylthio]propionamidin und die physiologisch annehmbaren Salze davon herstellt.

11. Verfahren nach Anspruch 1, dadurch gekennzeichnet, dass man N-Sulfamo-4-[(3-N,N-dimethylaminomethyl)phenoxy]butyroamidin und die physiologisch annehmbaren Salze davon herstellt.

12. Verfahren nach Anspruch 1, dadurch gekennzeichnet, dass man N-Sulfamoyl-4-[3-piperidinylmethyl)phenoxy]butyroamidin und die physiologisch annehmbaren Salze davon herstellt.

13. Verfahren nach Anspruch 1, dadurch gekennzeichnet, dass man N-Sulfamoyl-5-[(3-piperidinylmethyl)phenoxy]valeroamidin und die physiologisch annehmbaren Salze davon herstellt.

14. Verfahren nach Anspruch 1, dadurch gekennzeichnet, dass man N-(p-Aminobenzolsulfonyl)-5-[(3-puperidinylmethyl)phenoxy]valeroamidin und die physiologisch annehmbaren Salze davon herstellt.

15. Verfahren nach Anspruch 1, dadurch gekennzeichnet, dass man N-Sulfamoyl-3-[(2-(1-piperidinylmethyl)thienyl-4)methylthio]propionamidin und die physiologisch annehmbaren Salze davon herstellt.

16. Verfahren nach Anspruch 1, dadurch gekennzeichnet, dass man N-(p-Aminobenzolsulfonyl)-3-[(2-(1-piperidinylmethyl)thienyl-4)methylthio]propionamidin und die physiologisch annehmbaren Salze davon herstellt.

17. Verfahren nach Anspruch 1, dadurch gekennzeichnet, dass man N-Sulfamoyl-[(2-piperidinylmethyl-thienyl-4)methylthio]propionamidin und die physiologisch annehmbaren Salze davon herstellt.

18. Verfahren nach Anspruch 1, dadurch gekennzeichnet, dass man N-(p-Aminobenzolsulfonyl)-3-[(2-piperidinylmethylthienyl-4)methylthio]propionamidin und die physiologisch annehmbaren Salze davon herstellt.

19. Verfahren nach Anspruch 1, dadurch gekennzeichnet, dass man N-(p-Aminobenzolsulfonyl)-3-[(2-piperidinylmethylthiazol-4-yl)methylthio]propionamidin und die physiologisch annehmbaren Salze davon herstellt.

**Claims for the Contracting States BE, CH, DE, FR, GB, IT, LI, LU, NL, SE**

1. Heterocyclic compounds corresponding to the general tautomeric formulae I:

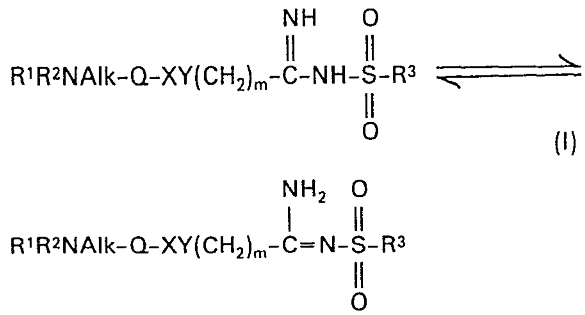

(I)

wherein $R^1$ stands for straight chained $C_{1-6}$alkyl and $R^2$ stands for staight chained $C_{1-6}$alkyl or $R^1$ and $R_2$ together with the nitrogen atom to which they are attached form a 5- to 6-membered ring;

Alk stands for a straight chained alkylene chain with 1 to 6 carbon atoms;

Q stands for a thiophene ring which is incorporated in the remainder of the molecule by bonds in the 2- and 5-position or in the 2- and 4-position, or Q stands for a benzene ring which is incorporated in the remainder of the molecule by bonds in the 1- and 3-position; when Q stands for thiophene then X stands for methylene, Y stands for sulphur and m has the value 2 or 3; when Q stands for benzene, then X stands for oxygen, Y stands for a single bond and m has the value 3 or 4; $R^3$ stands for an amino group or a p-aminophenyl group;

and the physiologically acceptable salts thereof.

2. Compounds according to claim 1, characterised in that $R^1$ stands for $C_{1-3}$ alkyl and $R^2$ stands for hydrogen, methyl or ethyl or $R^1R^2N$ denotes a 5- to 6-membered ring.

3. Compounds according to claim 1 or 2, characterised in that the goup Alk stands for methylene.

4. Compounds according to one of the claims 1 to 3, characterised in that Q stands for a thiophene ring which is incorporated in the remainder of the molecule by bonds in the 2- and 5-position and that the group $XY(CH_2)_m$ stands for $CH_2$-S-$(CH_2)_{2-3}$.

5. Compounds according to one of the claims 1 to 3, characterised in that Q stands for a thiophene ring which is incorporated in the remainder of the molecule by bonds in the 2- and 4-position and that the group $XY(CH_2)_m$ stands for $CH_2$-S-$(CH_2)_{2-3}$.

6. Compounds according to one of the claims 1 to 3, characterised in that Q stands for a benzene ring which is incorporated by bonds in the 1- and 3-position and that the group $XY(CH_2)_m$ stands for $O(CH_2)_{3-4}$.

7. N-(p-aminobenzenesulphonyl)-3-[(2-N,N-dimethylaminomethylthienyl - 4)methylthio]propionamidine and the physiologically acceptable salts thereof.

8. N-(p-aminobenzenesulphonyl)-4-[(3-N,N-dimethylaminomethyl)phenoxy]butyroamidine and the physiologically acceptable salts thereof.

9. N-(p-aminobenzenesulphonyl)-4-[3-(1-piperidinylmethyl)phenoxy]butyroamidine and the physiologically acceptable salts thereof.

10. N-sulphamoyl-3-[(2-N,N-dimethylaminomethylthienyl-4-)methylthio]propioamidine and the physiologically acceptable salts thereof.

11. N-sulphamoyl-4-[(3-N,N-dimethylaminomethyl)phenoxy]butyroamidine and the physiologically acceptable salts thereof.

12. N-sulphamoyl-4-[(3-piperidinylmethyl)-phenoxy]butyroamidine and the physiologically acceptable salts thereof.

13. N-sulphamoyl-5-[(3piperidinylmethyl)-phenoxy]valeroamidine and the physiologically acceptable salts thereof.

14. N-(p-aminobenzenesulphonyl)-5-[(3-piperidinylmethyl)phenoxy]valeroamidine and the physiologically acceptable salts thereof.

15. N-sulphamoyl-3-[(2(1-piperidinylmethyl)thienyl-4)methylthio]propionamidine and the physiologically acceptable salts thereof.

16. N-(p-aminobenzenesulphonyl)-3-[(2-(1-piperidinylmethyl)thienyl-4)methylthio]propionamidine and the physiologically acceptable salts therof.

17. N-sulphamoyl-[2-piperidinylmethylthienyl-4)methylthio]propionoamidine and the physiologically acceptable salts thereof.

18. N-(p-aminobenzenesulphonyl)-3-[(2-piperidinylmethylthienyl-4)methylthio]propionamidine and the physiologically acceptable salts thereof.

19. N-(p-aminobenzenesulphonyl)-3-[(2piperidinylmethyl-thiazol-4-yl)methylthio]propionoamidine and the physiologically acceptable salts thereof.

20. Process for the preparation of compounds according to claim 1, characterised in that a compound corresponding to the general formula II:

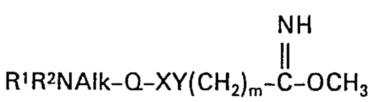

(II)

wherein $R^1$, $R^2$, Alk, Q, X, Y and m have the meanings indicated in claim 1 is reacted in known manner with a sulphonamide III:

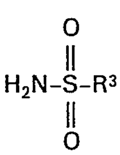

(III)

wherein $R^3$ has the meaning indicated in claim 1 and the compound obtained is optionally converted into its physiologically acceptable salt.

21. Therapeutic agent, characterised in that it contains a compound according to claim 1 and at least one inert, pharmaceutically acceptable carrier or an inert, pharmaceutically acceptable diluent, optionally together with at least one active ingredient.

**Claims for the Contracting State AT**

1. Process for the preparation of heterocyclic compounds corresponding to the general tautomeric formulae I:

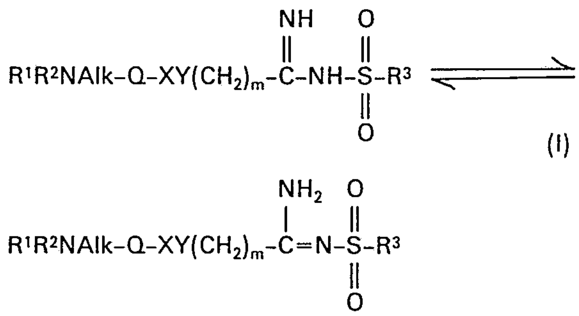

(I)

wherein $R^1$ stands for straight chained $C_{1-6}$ alkyl and $R^2$ stands for straight chained $C_{1-6}$ alkyl or $R^1$ and $R^2$ together with the nitrogem atom to which they are attached form a 5- or 6-membered ring; Alk stands for a straight chained alkylene chain with 1 to 6 carbon atoms;

Q stands for a thiopene ring which is incorporated in the remainder of the molecule by bonds in the 2- and 5-position or in the 2- and 4-position or Q stands for a benzene ring which is incorporated in the remainder of the molecule by bonds in the 1- and 3-position;

when Q denotes thiophene, X stands for methylene, Y stands for sulphur and m has the value 2 or 3; when Q denotes benzene, X stands for oxygen, Y stands for a single bond and m has the value 3 or 4; $R^3$ stands for an amino group or a p-aminophenyl group; and the physiologically acceptable salts thereof, characterised in that a compound corresponding to the general formula II:

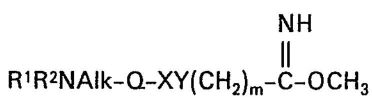

(II)

wherein $R^1$, $R^2$, Alk, Q, X, Y and m have the meanings indicated above is reacted in known manner with a sulphonamide III:

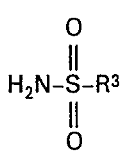

(III)

wherein $R^3$ has the meaning indicated above, and the resulting compound is optionally converted into its physiologically acceptable salt.

2. Process according to claim 1, characterised

in that $R^1$ stands for $C_{1-3}$ alkyl and $R^2$ stands for hydrogen, methyl or ethyl, of $R^1R^2N$ denotes a 5- to 6-membered ring.

3. Process according to claim 1 or 2, characterised in that the group Alk stands for methyl.

4. Process according to one of the claims 1 to 3, characterised in that Q stands for a thiophene ring which is incorporated in the remainder of the molecule by bonds in the 2- and 5- position and in that the group XY $(CH_2)_m$ stands for $CH_2$–S–$(CH_2)_{2-3}$.

5. Process according to one of the claims 1 to 3, characterised in that Q stands for a thiophene or thiazole ring which is incorporated in the remainder of the molecule by bonds in the 2- and 4-position and in that the group XY $(CH_2)_m$ stands for $CH_2$–S–$(CH_2)_{2-3}$.

6. Process according to one of the claims 1 to 3, characterised in that Q stands for a benzene ring which is incorporated by bonds in the 1- and 3-position and in that the group XY$(CH_2)_m$ stands for $O(CH_2)_{3-4}$.

7. Process according to claim 1, characterised in that N-(p-aminobenzenesulphonyl)-3-[(2-N,N-dimethylaminomethylthienyl-4) methylthio]propionamidine and the physiologically acceptable salts thereof are prepared.

8. Process according to claim 1, characterised in that N-(p-aminobenzenesulphonyl)-4-(3-N,N-dimethylaminomethyl)phenoxy]butyroamidine and the physiologically acceptable salts thereof are prepared.

9. Process according to claim 1, characterised in that N-(p-aminobenzenesulphonyl)-4-[3-(1-piperidinylmethyl)phenoxy]butyroamidine and the physiologically acceptable salts thereof are prepared.

10 Process according to claim 1, characterised in that N-sulphamoyl-3-[(2-N,N-dimethylaminomethylthienyl-4)methylthio]propionamidine and the physiologically acceptable salts thereof are prepared.

11. Process according to claim 1, characterised in that N-sulphamoyl-4-[(3-N,N-dimethylaminomethyl)phenoxy butyroamidine and the physiologically acceptable salts thereof are prepared.

12. Process according to claim 1, characterised in that N-sulphamoyl-4-(3-piperidinylmethyl)phenoxy]butyroamidine and the physiologically acceptable salts thereof are prepared.

13. Process according to claim 1, characterised in that N-sulphamoyl-5-[(3-piperidinylmethyl)-phenoxy]valeroamidine and the physiologically acceptable salts thereof are prepared.

14. Process according to claim 1, characterised in that N-(p-aminobenzenesulphonyl)-5-[(3-piperidinylmethyl)-phenoxy]valeroamidine and the physiologically acceptable salts thereof are prepared.

15. Process according to claim 1, characterised in that N-sulfamoyl-3-[(2-(1-piperidinylmethyl)thienyl-4)methylthio]propionamidine and the physiologically acceptable salts thereof are prepared.

16. Process according to claim 1, characterised in that N-(p-aminobenzenesulphonyl)-3-[(2-(1-piperidinylmethyl)thienyl-4)methylthio]-propionamidine and the physiologically acceptable salts thereof are prepared.

17. Process according to claim 1, characterised in that N-sulphamoyl-[(2-piperidinylmethyl-4)methylthio] propionamidine and the physiologically acceptable salts thereof are prepared.

18. Process according to claim 1, characterised in that N-(p-aminobenzenesulphonyl)-3-[(2-piperidinylmethylthienyl-4)methylthio]propionamidine and the physiologically acceptable salts thereof are prepared.

19. Process according to claim 1, characterised in that N-(p-aminobenzenesulphonyl)-3[(2-piperidinylmethylthiazol-4-yl)methylthio]-propionamidine and the physiologically acceptable salts thereof are prepared.

**Revendications pour les Etats contractants: BE, CH, DE, FR, GB, IT, LI, LU, NL, SE**

1. Composés hétérocycliques répondant aux formules tautomères générales I:

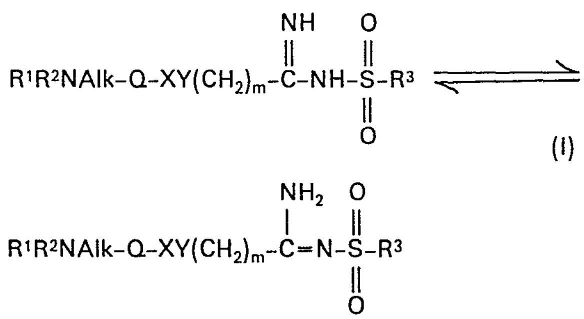

(I)

dans lesquelles $R^1$ représente un groupe alkyle linéaire de $C_1$ à $C_6$ et $R^2$ un groupe alkyle linéaire de $C_1$ à $C_6$, ou bien $R^1$ et $R^2$ représentent ensemble avec l'atome d'azote auquel ils sont liés un noyau à cinq ou six maillons;

Alk représente une chaîne alkylène linéaire comportant 1 à 6 atomes de carbone;

Q représente un noyau thiophène dont l'incorporation dans le reste de la molécule est effectuée par des liaisons en positions 2 et 5 ou en positions 2 et 4, ou bien Q représente un noyau benzène, dont l'incorporation dans le reste de la molécule est effectuée par des liaisons en positions 1 et 3; X représente, dans le cas où Q représente le thiophène, le méthylène, Y représente le soufre et m a pour valeur 2 ou 3; X représente, dans le cas où Q représente le benzène, l'oxygène, Y représente une liaison simple et m a pour valeur 3 ou 4; $R^3$ représente un groume amino ou un groupe p-aminophényle;

ainsi que leurs sels physiologiquement acceptables.

2. Composés selon la revendication 1, caractérisés en ce que $R^1$ représente un alkyle en $C_1$ à $C_3$

et $R^2$ l'hydrogène, un groupe méthyle ou un éthyle, ou bien $R^1R^2N$ représente un noyau à 5 à 6 maillons.

3. Composés selon la revendication 1 ou 2, caractérisés en ce que le groupe Alk représente le groupe méthylène.

4. Composés selon l'une des revendications 1 à 3, caractérisés en ce que Q représente un noyau thiophène dont l'incorporation dans le reste de la molécule est effectuée par des liaisons en positions 2 et 5, et en ce que le groupe $XY(CH_2)_m$ représente $CH_2{-}S{-}(CH_2)_{2-3}$.

5. Composés selon l'une des revendications 1 à 3, caractérisés en ce que Q représente un noyau thiophène dont l'incorporation dans le reste de la molécule est réalisée par des liaisons en positions 2 et 4, et en ce que le groupe $XY(CH)_2)_m$ représente $CH_2{-}S{-}(CH_2)_{2-3}$.

6. Composés selon l'une des revendications 1 à 3, caractérisés en ce que Q représente un noyau benzène incorporé par des liaisons en positions 1 et 3, et en ce que le groupe $XY(CH_2)_m$ représente $O(CH_2)_{3-4}$.

7. N-(p-aminobenzènesulfonyl)-3-[(2-N,N-diméthylaminométhylthiényl-4)méthylthio]propionamidine, et ses sels physiologiquement acceptables.

8. N-(p-aminobenzène-sulfonyl)-4-[(3-N,N-diméthylaminométhyl)phénoxy]-butyroamidine, et ses sels physiologiquement acceptables.

9. N-(p-aminobenzène-sulfonyl)-4-[3-(1-pipéridinylméthyl)-phénoxy]butyroamidine et ses sels physiologiquement acceptables.

10. N-sulfamyl-3-[(2-N,N-diméthylaminométhylthiényl-4)méthylthio]propionamidine et ses sels physiologiquement acceptables.

11. N-sulfamyl-4-[(3-N,N-diméthylaminométhyl)phénoxy]butyroamidine et ses sels physiologiquement acceptables.

12. N-sulfamyl-4-[(3-pipéridinylméthyl)phénoxy]butyroamidine et ses sels physiologiquement acceptables.

13. N-sulfamyl-5-[(3-pipéridinylméthyl)phénoxy]valéroamidine et ses sels physiologiquement acceptables.

14. N-(p-aminobenzène-sulfonyl)-5-[(3-pipéridinylméthyl)phénoxy]valéroamidine et ses sels physiologiquement acceptables.

15. N - sulfamyl - 3 - [(2 - (1 - pipéridinylméthyl) thiényl-4)méthylthio]propionamidine et ses sels physiologiquement acceptables.

16. N-(p-aminobenzène-sulfonyl)-3-[(2-(1-pipéridinylméthyl)thiényl-4)méthylthio]propionamidine et ses sels physiologiquement acceptables.

17. N-sulfamyl-[(2-pipéridinylméthylthiényl-4)méthylthio]propionamidine et ses sels physiologiquement acceptables.

18. N-(p-aminobenzène-sulfonyl)-3-[(2-pipéridinylméthylthiényl-4)méthylthio]propionamidine et ses sels physiologiquement acceptables.

19. N-(p-aminobenzène-sulfonyl)-3-[(2-pipéridinylméthylthiazol-4-yl)méthylthio]propionamidine et ses sels physiologiquement acceptables.

20. Procédé de préparation de composés selon la revendication 1, caractérisé en ce qu'on fait réagir un composé répondant à la formule générale II:

$$R^1R^2NAlk{-}Q{-}XY(CH_2)_m{-}\overset{\overset{\displaystyle NH}{\|}}{C}{-}OCH_3 \qquad (II)$$

dans laquelle $R^1$, $R^2$, Alk, Q, X, Y et m ont les significations indiquées dans la revendication 1, de façon connue en soi, sur un sulfonamide III:

$$H_2N{-}\overset{\overset{\displaystyle O}{\|}}{\underset{\underset{\displaystyle O}{\|}}{S}}{-}R^3 \qquad (III)$$

où $R^3$ a la signification indiquée dans la revendication 1, et l'on transforme éventuellement le composé obtenu en son sel physiologiquement acceptable.

21. Médicament, caractérisé en ce qu'il renferme un composé selon la revendication 1 et au moins un support inerte pharmaceutiquement acceptable, ou un diluant inerte pharmaceutiquement acceptable, ou un diluant inerte pharmaceutiquement acceptable, avec éventuellement au moins un autre agent actif.

**Revendications pour l'Etat contractant: AT**

1. Procédé de préparation de composés hétérocycliques répondant aux formules tautomères générales I:

$$R^1R^2NAlk{-}Q{-}XY(CH_2)_m{-}\overset{\overset{\displaystyle NH}{\|}}{C}{-}NH{-}\overset{\overset{\displaystyle O}{\|}}{\underset{\underset{\displaystyle O}{\|}}{S}}{-}R^3 \rightleftharpoons$$

$$R^1R^2NAlk{-}Q{-}XY(CH_2)_m{-}\overset{\overset{\displaystyle NH_2}{|}}{C}{=}N{-}\overset{\overset{\displaystyle O}{\|}}{\underset{\underset{\displaystyle O}{\|}}{S}}{-}R^3 \qquad (I)$$

dans lesquelles $R^1$ représente un groupe alkyle linéaire en $C_1$ à $C_6$ et $R^2$ un groupe alkyle linéaire en $C_1$ à $C_6$, ou bien $R^1$ et $R^2$ représentent ensemble, avec l'atome d'azote auquel ils sont liés, un noyau à 5 ou 6 maillons;

Alk représente une chaîne alkylène linéaire à 1 à 6 atomes de carbone;

Q représente un noyau thiophène dont l'incorporation dans le reste de la molécule est réalisée par des liaisons en positions 2 et 5 ou en positions 2 et 4, ou bien Q représente un noyau benzène dont l'incorporation dans le reste de la molécule est réalisée par des liaisons en positions 1 et 3; X représente, dans le cas où Q représente le thiophène, un groupe méthylène, Y représente le soufre et m a pour valeur 2 ou 3; X représente,

dans le cas où Q représente le benzène l'oxygène, Y représente une liaison simple et m a la valeur 3 ou 4; $R^3$ représente un groupe amino ou un groupe p-aminophényle;

ainsi que leurs sels physiologiquement acceptables, caractérisé en ce qu'on fait réagir un composé répondant à la formule générale II:

$$R^1R^2NAlk\text{-}Q\text{-}XY(CH_2)_m\text{-}\overset{\overset{NH}{\|}}{C}\text{-}OCH_3 \qquad (II)$$

dans laquelle $R^1$, $R^2$, Alk, Q, X, Y et m ont les significations indiquées précédemment, de façon connue en soi, sur un sulfonamide III

$$H_2N\text{-}\underset{\underset{O}{\|}}{\overset{\overset{O}{\|}}{S}}\text{-}R^3 \qquad (III)$$

où $R^3$ a la signification indiquée précédemment, et l'on transforme éventuellement le composé obtenu en son sel physiologiquement acceptable.

2. Procédé selon la revendication 1, caractérisé en ce que $R^1$ représente un groupe alkyle en $C_1$ à $C_3$ et $R^2$ représente l'hydrogène, un groupe méthyle ou un groupe éthyle, ou bien $R^1R^2N$ représente un noyau à 5 ou 6 maillons.

3. Procédé selon la revendication 1 ou 2, caractérisé en ce que le groupe Alk représente un groupe méthylène.

4. Procédé selon l'une des revendications 1 à 3, caractérisé en ce que Q représente un noyau thiophène dont l'incorporation dans le reste de la molécule est réalisée par des liaisons en positions 2 et 5, et en ce que le groupe $XY(CH_2)_m$ représente $CH_2\text{-}S\text{-}(CH_2)_{2\text{-}3}$.

5. Procédé selon l'une des revendications 1 à 3, caractérisé en ce que Q représente un noyau thiophène ou thiazole, dont l'incorporation dans le reste de la molécule est réalisée par des liaisons en positions 2 et 4, et en ce que le groupe $XY(CH_2)_m$ représente $CH_2\text{-}S\text{-}(CH_2)_{2\text{-}3}$.

6. Procédé selon les revendications 1 à 3, caractérisé en ce que Q représente un noyau benzène incorporé par des liaisons en positions 1 et 3, et en ce que le groupe $XY(CH_2)_m$ représente $O(CH_2)_{3\text{-}4}$.

7. Procédé selon la revendication 1, caractérisé en ce qu'on prépare la N-(p-aminobenzène-sulfonyl)-3-[(2,-N,N-diméthylaminométhylthiényl-4)méthylthio]propionamidine et ses sels physiologiquement acceptables.

8. Procédé selon la revendication 1, caractérisé en ce qu'on prépare de la N-(p-aminobenzène-sulfonyl)-4-[(3-N,N-diméthylaminométhyl) phénoxy]butyroamidine et ses sels physiologiquement acceptables.

9. Procédé selon la revendication 1, caractérisé en ce qu'on prépare la N-(p-aminobenzène-sulfonyl)-4-[3-(1-pipéridinylméthyl)phénoxy]butyroamidine et ses sels physiologiquement acceptables.

10. Procédé selon la revendication 1, caractérisé en ce qu'on prépare la N-sulfamyl-3-[(2-N,N-diméthylaminométhylthiényl-4)méthylthio]propionamidine et ses sels physiologiquement acceptables.

11. Procédé selon la revendication 1, caractérisé en ce qu'on prépare la N-sulfamyl-4-[(3-N,N-diméthylaminométhyl)phénoxy]butyroamidine et ses sels physiologiquement acceptables.

12. Procédé selon la revendication 1, caractérisé en ce qu'on prépare la N-sulfamyl-4-[(3-pipéridinylméthyl)-phénoxy]butyroamidine et ses sels physiologiquement acceptables.

13. Procédé selon la revendication 1, caractérisé en ce qu'on prépare la N-sulfamyl-5-[(3-pipéridinylméthyl)phénoxy]valéroamidine et ses sels physiologiquement acceptables.

14. Procédé selon la revendication 1, caractérisé en ce qu'on prépare la N-(p-aminobenzène-sulfonyl)-5-[(3-pipéridinylméthyl)phénoxy]valéroamidine et ses sels physiologiquement acceptables.

15. Procédé selon la revendication 1, caractérisé en ce qu'on prépare la N-sulfamyl-3-[(2-(1-pipéridinylméthyl)-thiényl-4)méthylthio]propionamidine et ses sels physiologiquement acceptables.

16. Procédé selon la revendication 1, caractérisé en ce qu'on prépare la N-(p-aminobenzène-sulfonyl)-3-[(2-(1-pipéridinylméthyl)thiényl-4)-méthylthio]propionamidine et ses sels physiologiquement acceptables.

17. Procédé selon la revendication 1, caractérisé en ce qu'on prépare la N-sulfamyl-[(2-pipéridinylméthylthiényl-4)méthylthio]propionamidine et ses sels physiologiquement acceptables.

18. Procédé selon la revendication 1, caractérisé en ce qu'on prépare la N-(p-aminobenzène-sulfonyl)-3-[(2-pipéridinylméthylthiényl-4)méthylthio]propionamidine et ses sels physiologiquement acceptables.

19. Procédé selon la revendication 1, caractérisé en ce qu'on prépare la N-(p-aminobenzène-sulfonyl)-3-[(2-pipéridinylméthylthiazol-4-yl)méthylthio]propionamidine et ses sels physiologiquement acceptables.